(19) Europäisches Patentamt
European Patent Office
Office européen des brevets

(11) Veröffentlichungsnummer: **0 093 977**
**B1**

(12) # EUROPÄISCHE PATENTSCHRIFT

(45) Veröffentlichungstag der Patentschrift: **05.08.87**

(21) Anmeldenummer: **83104197.5**

(22) Anmeldetag: **29.04.83**

(51) Int. Cl.⁴: **C 07 C 127/22,** C 07 C 149/34, A 01 N 47/34, C 07 D 319/20, C 07 C 147/06, C 07 C 143/02, C 07 D 317/46, C 07 C 149/32, C 07 D 319/08 // C07C157/12

(54) **2,5-Dihalogenbenzoyl-(thio)harnstoffe, Verfahren zu ihrer Herstellung und ihre Verwendung als Schädlingsbekämpfungsmittel.**

(30) Priorität: **11.05.82 DE 3217620**

(43) Veröffentlichungstag der Anmeldung: **16.11.83 Patentblatt 83/46**

(45) Bekanntmachung des Hinweises auf die Patenterteilung: **05.08.87 Patentblatt 87/32**

(84) Benannte Vertragsstaaten: **BE CH DE FR GB IT LI NL**

(56) Entgegenhaltungen:
**EP - A - 0 008 435**
**EP - A - 0 008 768**
**DE - A - 2 601 780**
**DE - A - 2 801 316**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhoevel (DE)**
Erfinder: **Klauke, Erich, Dr., Eichendorffweg 8, D-5068 Odenthal (DE)**
Erfinder: **Hammann, Ingeborg, Dr., Lutherstrasse 22, D-4330 Mülheim/Ruhr (DE)**

**EP 0 093 977 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2,5-Dihalogenbenzoyl-(thio)harnstoffe, mehrere Verfahren zu ihrer Herstellung und ihre Verwendung als Schädlingsbekämpfungsmittel, insbesondere als Insektizide.

Es ist bereits bekannt, dass bestimmte Benzoylharnstoffe, wie z.B.
1-(2,6-Dichlor-benzoyl)-3-(4-chlorphenyl)-harnstoff und
1-(2,6-Dichlor-benzoyl)-3-(3,4-dichlorphenyl)-harnstoff,
insektizide Eigenschaften besitzen, (vergleiche z.B. DE-AS 2 123 236 sowie die entsprechende US-PS 3 933 908 sowie US-PS 4 139 636), wobei die 2- und 2,6-substituierten Benzylharnstoffe und -thioharnstoffe als besonders insektizid wirksam beschrieben werden.

Es wurden die neuen 2,5-Dihalogenbenzoyl-(thio)harnstoffe der Formel (I)

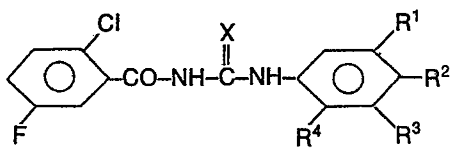 (I)

gefunden,
in welcher
X für Schwefel oder Sauerstoff steht,

$R^1$ für Wasserstoff, Halogen oder für einen gegebenenfalls durch Halogen substituierten Rest aus der Reihe $C_1$–$C_6$-Alkyl, $C_1$–$C_6$-Alkoxy und $C_1$–$C_6$-Alkylthio steht,

$R^2$ für Wasserstoff, Cyano, Nitro, Halogen oder für einen gegebenenfalls durch Halogen substituierten Rest aus der Reihe
$C_1$–$C_6$-Alkyl, $C_1$–$C_6$-Alkylthio,
$C_1$–$C_6$-Alkylsulfonyl, $C_1$–$C_6$-Alkoxy,
$C_2$–$C_6$-Alkylcarbonyl, $C_2$–$C_6$-Alkoxycarbonyl,
$C_3$–$C_6$-Alkoxycarbonylalkyl,
$C_3$–$C_6$-Alkoxycarbonylalkylthio
oder für einen gegebenenfalls durch
Halogen, Cyano, Nitro, Phenyl,
Trifluormethyl, Trifluormethoxy,
Trifluormethylthio, $C_1$–$C_6$-Alkyl,
$C_1$–$C_6$-Alkoxy, $C_1$–$C_6$-Alkylthio,
$C_1$–$C_6$-Alkylthioalkyl,
$C_1$–$C_6$-Alkylsulfonyloxy und/oder
$C_2$–$C_6$-Alkoxycarbonyl substituiertes Phenyloxy steht, oder

$R^1$ und $R^2$ gemeinsam für gegebenenfalls durch Fluor und/oder Chlor substituiertes Alkylen-dioxy mit 1 bis 3 Kohlenstoffatomen oder für $-CF_2-O-CF_2-O-$ stehen,

$R^3$ für Wasserstoff, Halogen oder für einen gegebenenfalls durch Halogen substituierten $C_1$–$C_6$-Alkyl-, $C_1$–$C_6$-Alkoxy- oder Phenyloxy-Rest steht, und

$R^4$ für Wasserstoff, Halogen oder für gegebenenfalls durch Halogen substituierte Reste aus der Reihe $C_1$–$C_6$-Alkyl, $C_1$–$C_6$-Alkylthio oder $C_1$–$C_6$-Alkoxy steht.

Diese neuen Verbindungen weisen starke biologische, insbesondere insektizide Eigenschaften aus, die ihre Verwendung als Schädlingsbekämpfungsmittel, insbesondere als Insektizide ermöglichen.

Weiterhin wurde gefunden, dass die neuen 2,5-Dihalogenbenzoyl-Harnstoffe der Formel (I) erhalten werden, indem man

a) substituierte Aniline der Formel (II)

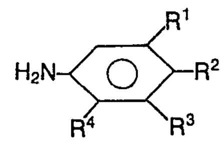 (II)

in welcher
$R^1$, $R^2$, $R^3$ und $R^4$ die oben angegebenen Bedeutungen haben,
mit Benzoyl-iso(thio)cyanaten der Formel (III)

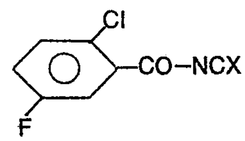 (III)

in welcher
X die oben angegebene Bedeutung hat,
gegebenenfalls in Gegenwart eines Verdünnungsmittels umsetzt oder

b) substituierte Phenyl-iso(thio)cyanate der Formel (IV)

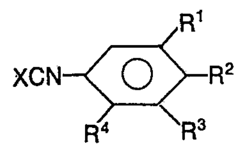 (IV)

in welcher
$R^1$, $R^2$, $R^3$, $R^4$ und X die oben angegebenen Bedeutungen haben,
mit Benzoesäureamid der Formel (V)

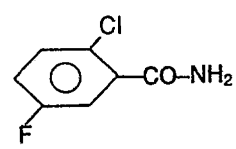 (V)

gegebenenfalls in Gegenwart eines Katalysators und gegebenenfalls in Gegenwart eines Verdünnungsmittels umsetzt.

Als gegebenenfalls substituiertes Alkyl $R^1$, $R^2$, $R^3$ und $R^4$ steht geradkettiges oder verzweigtes Alkyl mit 1 bis 6, insbesondere 1 bis 4 Kohlenstoffatomen. Beispielhaft seien gegebenenfalls substituiertes Methyl, Ethyl, n- und i-Propyl, n-, i-, sec- und tert.Butyl genannt.

Als gegebenenfalls substituiertes Alkoxy $R^1$, $R^2$, $R^3$ und $R^4$ steht geradkettiges oder verzweigtes Alkoxy mit 1 bis 6, insbesondere 1 bis 4 Kohlenstoffatomen. Beispielhaft seien gegebenenfalls substituiertes Methoxy, Ethoxy, n- und i-Propoxy und n-, i-, sec- und tert.-Butoxy genannt.

Als gegebenenfalls substituiertes Alkylthio $R^1$, $R^2$, $R^4$ und Alkylsulfonyl $R^2$ steht geradkettiges oder verzweigtes Alkylthio oder Alkylsulfonyl mit 1 bis 6, insbesondere 1 bis 4 Kohlenstoffatomen.

Beispielhaft seien gegebenenfalls substituiertes Methylthio, Ethylthio, n- und i-Propylthio, n-, i-, sec-, tert.-Butylthio, Methylsulfonyl, Ethylsulfonyl, n- und i-Propylsulfonyl, n-, i-, sec- und tert.-Butylsulfonyl genannt.

Gegebenenfalls substituiertes Alkylcarbonyl, Alkoxycarbonyl, Alkoxycarbonylalkyl oder Alkoxycarbonylalkylthio $R^2$ entspricht in seinem Alkylteil dem Alkylrest $R^2$, in seinem Alkoxyteil dem Alkoxyrest $R^2$ und in seinem Alkylthioteil dem Alkylthiorest $R^2$.

Gegebenenfalls substituiertes Alkylendioxy in der Definition von $R^1$ und $R^2$ enthält 1 bis 3, insbesondere 1 oder 2 Kohlenstoffatome.

Halogen bedeutet (wo nicht anders erläutert) Fluor, Chlor, Brom und Jod, vorzugsweise Fluor, Chlor und Brom.

Die Reste $R^1$, $R^2$, $R^3$ und $R^4$ können einfach oder mehrfach, gleich oder verschieden substituiert sein.

Im Falle von Halogen sind die Halogenatome gleich oder verschieden und bedeuten vorzugsweise Fluor, Chlor oder Brom, insbesondere Fluor und Brom.

X steht bevorzugt für Sauerstoff.

Die neuen Verbindungen der Formel (I) verfügen über Eigenschaften, die ihre Verwendung als Schädlingsbekämpfungsmittel ermöglichen, insbesondere zeichnen sie sich durch eine hervorragende insektizide Wirksamkeit aus.

Bevorzugt sind die Verbindungen der Formel (I), in welcher

X für Sauerstoff oder Schwefel steht,

$R^1$ für Wasserstoff, Fluor, Chlor oder Brom steht,

$R^2$ für Wasserstoff, Chlor, Brom, Fluor, Cyano, Nitro, Methyl, Ethyl, n-Propyl, i-Propyl, n-Butyl, i-Butyl, sec.Butyl, tert.Butyl, Trifluormethyl, Difluormethyl, 1,1-Difluorethyl, 1,1,2,2,2-Pentafluorethyl, Trifluormethoxy, Difluormethoxy, Chlordifluormethoxy, 2-Chlor-1,1,2-trifluorethoxy, 1,1,2,2-Tetrafluorethoxy, 2,2-Dichlor-1,1-difluorethoxy, 1,1,2,3,3,3-Hexafluorpropoxy, Trifluormethylthio, Difluormethylthio, Chlordifluormethylthio, 2-Chlor-1,1,2-trifluorethylthio, Methoxycarbonyldifluormethylthio, 1,1,2,3,3,3-Hexafluorpropylthio, Trifluormethylsulfonyl, Methoxy-, Ethoxy-, n-Propoxy-, i-Propoxy-, n-Butoxy-, i-Butoxy-, sec-Butoxy- und tert. Butoxycarbonyl oder für gegebenenfalls durch Halogen, Nitro, Cyano, $C_1$–$C_6$-Halogenalkyl oder $C_1$–$C_4$-Alkoxycarbonyl substituiertes Phenyloxy steht, oder

$R^1$ und $R^2$ gemeinsam für Difluormethylendioxy, für $-CF_2-O-CF_2-O-$ oder für Ethylendioxy stehen, welches durch 3 oder 4 Fluoratome oder durch 3 Fluoratome und 1 Chloratom substituiert ist,

$R^3$ für Wasserstoff, Fluor, Chlor oder Brom steht, und

$R^4$ für Wasserstoff, Chlor, Brom, Trifluormethyl, Trifluormethoxy und Trifluormethylthio steht.

Besonders bevorzugt sind die Verbindungen der Formel (I), in welcher

X für Sauerstoff steht,

$R^1$ für Wasserstoff oder Chlor steht,

$R^2$ für Wasserstoff, Triflourmethyl, Trifluormethoxy, Difluormethoxy, Chloridfluormethoxy, Chlortrifluorethoxy, n-Pentafluorpropoxy, Trifluormethylthio, Chlordifluormethylthio, tert.Butoxycarbonyl, 4-Nitrophenoxy, 4-Cyanophenoxy und 4-Trifluormethylphenoxy steht, oder

$R^1$ und $R^2$ gemeinsam für Chlortrifluorethylendioxy oder für $-CF_2-O-CF_2-O-$ stehen,

$R^3$ für Wasserstoff oder Chlor steht, und

$R^4$ für Wasserstoff steht.

Verwendet man nach Verfahrenvariante (a) 3-Chlor-4-trifluor-methoxyanilin und 2-Chlor-5-fluorbenzoylisocyanat als Ausgangsstoffe, so kann der Reaktionsablauf durch das folgende Formelschema wiedergegeben werden:

a) 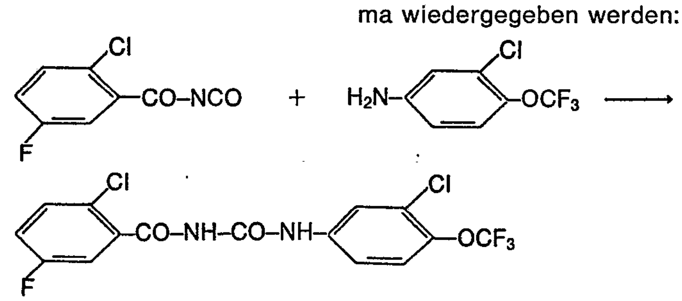

Verwendet man nach Verfahrenvariante (b) 3-Chlor-4-trifluormethoxy-phenylisocyanat und 2-Chlor-5-fluor-benzamid als Ausgangsstoffe, so kann der Reaktionsablauf durch das folgende Formelschema wiedergegeben werden:

b) 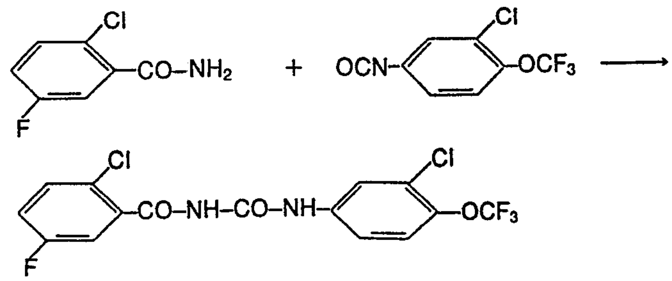

Als Beispiele für die Verbindungen der Formel (II) seien genannt:
4-Trifluormethoxy-,
3,4-Chlortrifluorethylendioxy-,
4-Trifluormethylthio-,
3-Chlor-4-chlordifluormethylthio-,
3-Chlor-4-chlortrifluorethoxy-,
4-Chlor-, 4-Trifluormethyl-, 3,4-Dichlor-,
4-Pentafluorpropoxy-, 4-Methyl-, 4-Ethyl-,
4-n-Propyl-, 4-i-Propyl-, 4-n-Butyl-,
4-i-Butyl-, 4-sec.Butyl-, 4-tert.Butyl-,
3,5-Dichlor-, 4-Chlordifluormethoxy-,
3-Chlor-4-trifluormethoxy-,
4-Chlortrifluorethoxy,
3-Chlor-4-trifluormethylthio,
4-Brom-, 2-Brom-, 3-Chlor-4-nitrophenoxy-,
3,5-Dichlor-4-(4-nitrophenoxy)-,
3,4,6-Trichlor-, 2-Chlor-,
3-Chlor-4-trifluormethyl-, 2-Trifluormethoxy-,
3,5-Dichlor-4-(4-cyanophenoxy)-,
3,5-Dichlor-4-(4-trifluormethylphenoxy)-,
3,5-Dichlor-4-(2-chlor-4-trifluormethylphenoxy)- und 4-Fluor-anilin.

Die als Ausgangsstoffe zu verwendenden substituierten Aniline der Formel (II) sind bekannt und nach literaturbekannten Verfahren und Methoden herstellbar (vergleiche z.B. DE-OS 2 601 780, DE-OS 2 801 316, DE-OS 2 837 524, DE-OS 2 843 851 und DE-OS 3 023 328).

Als Beispiele für die Verbindungen der Formel (III) seien genannt:
2-Chlor-5-fluor-benzoylisocyanat bzw. -benzoyl-isothiocyanat.

Die Ausgangsverbindungen der Formel (III) sind bekannt.

Als Beispiele für die Verbindungen der Formel (IV) seien genannt:
4-Trifluormethoxy-, 4-Trifluormethyl-,
4-Trifluormethylthio-, 3-Chlor-4-trifluormethoxy-,
3-Chlor-4-trifluormethyl-,
3-Chlor-4-trifluormethylthio-,
3,4-Chlortrifluorethylendioxy-,
3-Chlor-4-chlordifluormethylthio-,
3-Chlor-4-chlortrifluorethoxy-,
4-Chlor-, 2-Chlor-, 3,4-Dichlor-,
4-Pentafluorpropoxy-, 4-Methyl-, 4-Ethyl-,
4-n-Propyl-, 4-i-Propyl-, 4-n-Butyl-,
4-i-Butyl-, 4-sec.Butyl-, 4-tert.Butyl-,
3,5-Dichlor-, 4-Chlordifluormethoxy-,
3-Chlor-4-trifluormethoxy-,
4-Chlortrifluorethoxy-,
3-Chlor-4-trifluormethylthio-, 2-Brom-,
4-Brom-, 3-Chlor-4-nitrophenoxy-,
3-5-Dichlor-4-(4-nitrophenoxy)-,
3,4,6-Trichlor-, 2-Trifluormethoxy-,
3,5-Dichlor-4-(4-cyanophenoxy)-,
3,5-Dichlor-4-(4-trifluormethylphenoxy)-,
3,5-Dichlor-4-(2-chlor-4-trifluormethylphenoxy)- und 4-Fluorphenyl-iso(thio)cyanat.

Die Verbindungen der Formel IV sind bekannt oder können nach allgemein bekannten Verfahren und Methoden hergestellt werden.

Als Beispiel für die Verbindungen der Formel (V) sei genannt:
2-Chlor-5-fluor-benzoesäureamid.

Die Verbindung der Formel (V) ist bekannt und kann nach bekannten Methoden hergestellt werden (vergl. z.B. J. Am. Chem. Soc. 81, 94 (1959).

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methylpyrrolidon sowie Tetramethylensulfon.

Als Katalysatoren können für die Umsetzung gemäss Verfahrensvariante (b) vorzugsweise tertiäre Amine, wie Triethylamin und 1,4-Diazabicyclo[2,2,2]-octan, sowie organische Zinn-Verbindungen, wie z.B. Dibutylzinndilaurat verwendet werden.

Die Reaktionstemperatur kann innerhalb eines grösseren Bereiches variiert werden. Im allgemeinen arbeitet man bei der Verfahrensvariante a) zwischen 20 und 180 °C, vorzugsweise zwischen 60 und 120 °C und bei den Verfahrensvarianten b) zwischen 20 und 200 °C, vorzugsweise zwischen 60 und 190 °C. Die erfindungsgemässen Verfahrensvarianten werden im allgemeinen bei Normaldruck durchgeführt.

Zur Durchführung der erfindungsgemässen Verfahrensvarianten werden die Ausgangsstoffe gewöhnlich in etwa äquimolaren Mengen eingesetzt. Ein Überschuss der einen oder anderen Reaktionskomponente bringt keine wesentlichen Vorteile.

Die Aufarbeitung der Reaktionsprodukte geschieht nach üblichen Methoden z.B. durch Absaugen des angefallenen Produktes oder durch Herauslösen von unerwünschten Nebenprodukten aus dem Reaktionsgemisch. Zur Charakterisierung dient der Schmelzpunkt.

Die Wirkstoffe eignen sich bei guter Pflanzenverträglichkeit zur Bekämpfung von tierischen Schädlingen, insbesondere Insekten, die in der Landwirtschaft, im Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.

Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana, Tineola bisselliella, Tinea pellionella, Hofmannophila pseudospretella.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus supp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die neuen Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder fester Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: stark polare Lösungsmittel, wie Dimethylformamid und Dimethylsulfoxid; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-Äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Auch der Formel I entsprechende Verbindungen mit Anilin-Resten, wie sie in den Verbindungen enthalten sind, welche in DE-AS 1 123 236 und 3 041 957; EP-OS 6184, 7687, 8768, 13 414, 14 674, 14 675, 14 676, 16 729, 23 884, 25 363, 30 518, 31 974, 33 231, 35 084, 38 776, 40 179, 44 278 und 44 410 sowie JP-PS 55 011 537, 56 015 272 und 56 092 857 beschrieben wurden, weisen als 2,5-Dihalogenbenzoyl(thio)harnstoff-Derivate eine insektizide Wirksamkeit auf.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten (mit Kalk gestrichenen) Unterlagen aus.

Die erfindungsgemässen Wirkstoffe eignen sich auch zur Bekämpfung von Ekto- und Endoparasiten auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemässen Wirkstoffe geschieht auf diesen Gebieten in bekannter Weise, wie durch äusserliche Anwendung in Form beispielsweise des Tauchens (Dippen), Sprühens (Sprayen), Aufgiessens (pour-on and spot-on) und des Einpuderns sowie durch orale Anwendung beispielsweise über das Futter oder Trinkwasser in Form von beispielsweise Tabletten, Kapseln, Tränken, Granulaten.

Die Wirksamkeit der erfindungsgemässen Wirkstoffe sei anhand der folgenden Beispiele erläutert.

Beispiel A

Plutella-Test

| | |
|---|---|
| Lösungsmittel: | 3 Gewichtsteile Dimethylformamid |
| Emulgator: | 1 Gewichtsteil Alkylarylpolyglykoläther |

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden. (Kontrolle)

Bei diesem Test zeigten z.B. bei einer Konzentration von 0,001% nach 7 Tagen beispielsweise die Verbindungen der Herstellungsbeispiele (4), (6), (7), (9), (14), (16), (24), (27), (30), (32), (33), (36), (38), (39), (40), (42), (47) und (50) einen Abtötungsgrad von 100%.

Beispiel B

Laphygma-Test

| | |
|---|---|
| Lösungsmittel: | 3 Gewichtsteile Dimethylformamid |
| Emulgator: | 1 Gewichtsteil Alkylarylpolyglykoläther |

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% (Kontrolle) bedeutet, dass keine Raupen getötet wurden.

Bei diesem Test zeigte z.B. bei einer Wirkstoffkonzentration von 0,001% nach 7 Tagen beispielsweise die Verbindungen der Herstellungsbeispiele (1), (2) und (9) einen Abtötungsgrad von 100%.

Die Herstellung der erfindungsgemässen Verbindungen soll anhand der folgenden Beispiele erläutert werden:

Beispiel 1

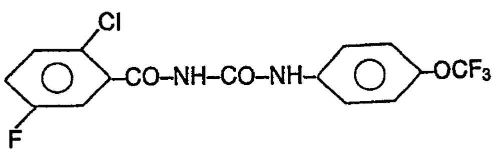

(Verfahrensvariante a)

Zu 2,66 g (0,015 Mol) 4-Trifluormethoxyanilin in 30 ml trockenem Toluol fügt man unter Feuchtigkeitsausschuss bei 60 °C 2,99 g (0,015 Mol) 2-Chlor-5-fluor-benzoylisocyanat. Der Ansatz wird eine Stunde bei 80 °C gerührt und auf 20–25 °C abgekühlt. Das ausgefallene Produkt wird abgesaugt und im Vakuum bei 100 °C getrocknet. Man erhält 5,6 g (100% der Theorie) 1-(2-Chlor-5-fluorbenzoyl)-3-(4-trifluormethoxyphenyl)-harnstoff vom Schmelzpunkt 199 °C.

(Verfahrensvariante b)

Zu 3,48 g (0,02 Mol) 2-Chlor-5-fluorbenzamid in 100 ml trockenem Toluol fügt man bei 100 °C unter Feuchtigkeitsausschuss eine Lösung von 4,06 g (0,02 Mol) 4-Trifluormethoxy-phenylisocyanat in 10 ml trockenem Toluol. Der Ansatz wird 10 Stunden unter Rückfluss gekocht. Nach dem Abkühlen wird das ausgefallene Produkt abgesaugt und mit Methanol nachgewaschen. Man erhält 6,2 g (82,5% der Theorie) 1-(2-Chlor-5-fluor-benzoyl)-3-(4-trifluormethoxyphenyl)-harnstoff vom Schmelzpunkt 199 °C. Die Verbindung ist mit der nach Verfahrensvariante (a) hergestellten Verbindung identisch.

Analog Beispiel 1 bzw. Verfahrensvarianten (a) und (b) können die in der nachstehenden Tabelle aufgeführten Verbindungen der Formel (Ia) hergestellt werden:

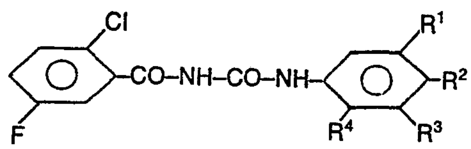 (Ia)

Tabelle 1

| Beisp. Nr. | $R^1$ | $R^2$ | $R^3$ | $R^4$ | Schmelzpunkt/(°C) |
|---|---|---|---|---|---|
| 2 | H | $CF_3$ | H | H | 225 |
| 3 | H | Cl | H | H | 206 |
| 4 | H | $SCF_3$ | H | H | 176 |
| 5 | H | tert.-$C_4H_9$ | H | H | 193 |
| 6 | H | $COOC_4H_9$-tert. | H | H | 185 |
| 7 | Cl | $COOC_4H_9$-tert. | H | H | 207 |
| 8 | H | $OCHF_2$ | H | H | 175 |
| 9 | Cl | 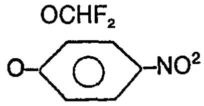 | Cl | H | 212 |
| 10 | H | )–$CF_2$–$CHCl_2$ | H | H | 173 |
| 11 | Cl | Cl | H | H | 203 |
| 12 | H | Br | H | H | 200 |
| 13 | H | F | H | H | 184 |
| 14 | Cl | H | Cl | H | 169 |
| 15 | H | $NO_2$ | H | H | 226 |
| 16 | Cl | 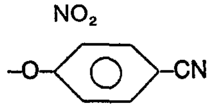 | Cl | H | 217 |
| 17 | $CF_3$ | H | $CF_3$ | H | 138 |
| 18 | –O–$CH_2$–$CF_2$–O– | | H | H | 183 |
| 19 | $CF_3$ | H | H | $CF_3$ | 114 |
| 20 | $CF_3$ | $CF_3$ | H | H | 164 |
| 21 | $CF_3$ | H | H | H | 166 |
| 22 | $CF_3$ | $OCH_3$ | H | H | 191 |
| 23 | $CF_3$ | Cl | H | H | 173 |
| 24 | Cl | 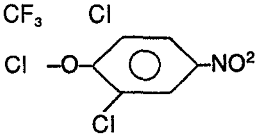 | Cl | H | 213 |

Tabelle 1 (Fortsetzung)

| Beisp. Nr. | $R^1$ | $R^2$ | $R^3$ | $R^4$ | Schmelz-punkt/(°C) |
|---|---|---|---|---|---|
| 25 | $CF_3$ | F | H | H | 159 |
| 26 | H | 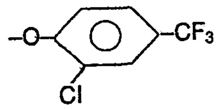 | H | H | 156 |
| 27 | H | $SO_2CF_3$ | H | H | 202 |
| 28 | H | 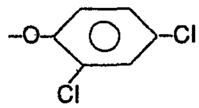 | H | H | 187 |
| 29 | H | 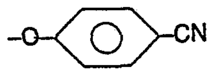 | Cl | H | 202 |
| 30 | H | 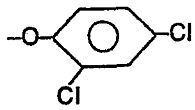 | Cl | H | 142 |
| 31 | Cl | 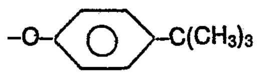 | Cl | H | 190 |
| 32 | $CH_3$ | 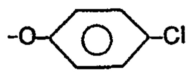 | $CH_3$ | H | 177 |
| 33 | $CH_3$ | 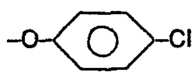 | Cl | H | 180 |
| 34 | H | 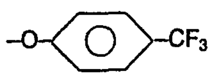 | H | H | 172 |
| 35 | $CF_3$ | $SCH_3$ | H | H | 157 |
| 36 | Cl | $SCF_2Cl$ | H | H | 144 |
| 37 | Cl | $SCF_3$ | H | H | 173 |
| 38 | Cl | $-OCF_2-CHFCl$ | H | H | 168 |
| 39 | H | $-OCF_2CHF-CF_3$ | H | H | 177 |
| 40 | H | $OCF_2Cl$ | H | H | 191 |
| 41 | $SCF_3$ | H | H | H | 137 |
| 42 | $-O-CFCl-CF_2-O-$ | | H | H | 172 |
| 43 | Cl | 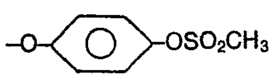 | Cl | H | 197 |
| 44 | Cl | 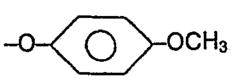 | Cl | H | 209 |
| 45 | H | H | H | $OCF_3$ | 125 |
| 46 | $-O-CF_2-O-$ | | H | Cl | 206 |
| 47 | $-O-CHF-CF_2-O-$ | | H | H | 194 |
| 48 | $-O-CF_2-O-$ | | H | H | 189 |
| 49 | $OCHF_2$ | $OCHF_2$ | H | H | 177 |
| 50 | Cl | $OCF_2-CHF_2$ | H | H | 171 |
| 51 | Cl | 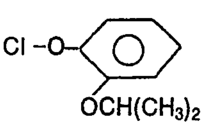 | Cl | H | 146 |

Tabelle 1 (Fortsetzung)

| Beisp. Nr. | $R^1$ | $R^2$ | $R^3$ | $R^4$ | Schmelz-punkt/(°C) |
|---|---|---|---|---|---|
| 52 | Cl | 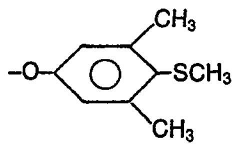 | Cl | H | 234 |
| 53 | Cl | 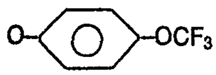 | Cl | H | 187 |
| 54 | Cl | $-CO-OCH_2CF_3$ | H | H | 159 |
| 55 | Cl | 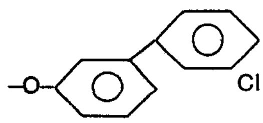 | Cl | H | 170 |
| 56 | H | $-CO-OCH_2CF_3$ | H | H | 175 |
| 57 | $CF_3$ | $-OCHF_2$ | H | H | 151 |
| 58 | H | $-OCF_2CHFCl$ | H | H | 190 |
| 59 | H | $-CF_3$ | H | Cl | 203 |
| 60 | Cl | 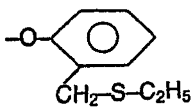 | Cl | H | 192 |
| 61 | Cl | 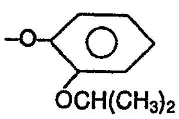 | H | H | 132 |
| 62 | Cl | 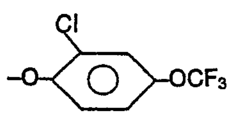 | Cl | H | 169 |
| 63 | $-CF_2-O-CF_2-O$ | | H | H | 184 |

Die Herstellung der Ausgangsverbindungen der Formel V kann beispielsweise wie folgt durchgeführt werden:

In einem 5 l-Rührkessel mit Kühler (–10 °C) werden 1,8 l wasserfreie Flusssäure vorgelegt, bei –5 °C bis 0 °C werden dann portionsweise 726 g, 2-Chlor-5-amino-benzoesäure eingetragen, anschliessend trägt man bei 0–5 °C 480 g $NaNO_2$ ebenfalls portionsweise ein, lässt 30 Min. nachrühren und gibt dann 800 ml DMSO hinzu. Man lässt bei 80–85 °C bis zum Ende der $N_2$-Abspaltung ausreagieren. Der Ansatz wird abgekühlt und auf ca. 5 kg Eis gegossen, abgesaugt, der Filterrückstand alkalisch gelöst, filtriert und mit HCl wieder ausgefällt, abgesaugt, der Filterrückstand nochmals in $CH_2Cl_2$ gelöst (um vom restlichen NaF zu befreien), filtriert und wieder eingeengt.

Ausbeute: 463 g Fp: 145 – 6 °C.

In vorgelegtes überschüssiges Thionylchlorid werden 350 g obiger Säure eingetragen. Nach Ende der Zugabe und der HCl-Entwicklung wird bis ca. 100 °C geheizt und nach Beendigung der Reaktion durch Destillation aufgearbeitet.

Ausbeute: 317 g Siedepunkt 103 °C/24 mbar $n_D^{20}$: 1.5487

Durch Umsetzung mit überschüssiger ca. 12,5% $NH_3$-Lösung erhält man nach Filtration 258 g 2-Chlor-5-fluor-benzamid Fp: 138 °C.

**Patentansprüche**

1. 2,5-Dihalogenbenzoyl-(thio)harnstoffe der Formel (I)

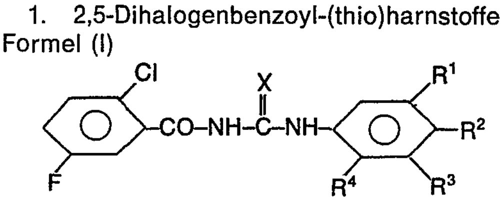 (I)

in welcher

X für Schwefel oder Sauerstoff steht,

$R^1$ für Wasserstoff, Halogen oder für einen gegebenenfalls durch Halogen substituierten Rest aus der Reihe $C_1$–$C_6$-Alkyl, $C_1$–$C_6$-Alkoxy und $C_1$–$C_6$-Alkylthio steht,

$R^2$ für Wasserstoff, Cyano, Nitro, Halogen oder für einen gegebenenfalls durch Halogen substituierten Rest aus der Reihe $C_1$–$C_6$-Alkyl, $C_1$–$C_6$-Alkylthio, $C_1$–$C_6$-Alkylsulfonyl, $C_1$–$C_6$-Alkoxy, $C_2$–$C_6$-Alkylcarbonyl, $C_2$–$C_6$-Alkoxycarbonyl, $C_3$–$C_6$-Alkoxycarbonylalkyl, $C_3$–$C_6$-Alkoxycarbonylalkylthio oder für einen gegebenenfalls durch Halogen, Cyano, Nitro, Phenyl, Trifluormethyl, Trifluormethoxy, Trifluormethylthio, $C_1$–$C_6$-Alkyl, $C_1$–$C_6$-Alkoxy, $C_1$–$C_6$-Alkylthio, $C_1$–$C_6$-Alkylthioalkyl, $C_1$–$C_6$-Alkylsulfonyloxy und/oder $C_2$–$C_6$-Alkoxycarbonyl substituiertes Phenyloxy steht, oder

$R^1$ und $R^2$ gemeinsam für gegebenenfalls durch Fluor und/oder Chlor substituiertes Alkylen-dioxy mit 1 bis 3 Kohlenstoffatomen oder für $-CF_2-O-CF_2-O-$ stehen,

$R^3$ für Wasserstoff, Halogen oder für einen gegebenenfalls durch Halogen substituierten $C_1$–$C_6$-Alkyl-, $C_1$–$C_6$-Alkoxy- oder Phenyloxy-Rest steht, und

$R^4$ für Wasserstoff, Halogen oder für gegebenenfalls durch Halogen substituierte Reste aus der Reihe $C_1$–$C_6$-Alkyl, $C_1$–$C_6$-Alkylthio oder $C_1$–$C_6$-Alkoxy steht.

2. Verbindungen gemäss Anspruch 1, wobei

X für Sauerstoff oder Schwefel steht,

$R^1$ für Wasserstoff, Fluor, Chlor oder Brom steht,

$R^2$ für Wasserstoff, Chlor, Brom, Fluor, Cyano, Nitro, Mehtyl, Ethyl, n-Propyl, i-Propyl, n-Butyl-, i-Butyl, sec.-Butyl, tert.-Butyl, Trifluormethyl, Difluormethyl, 1,1-Difluorethyl, 1,1,2,2,2-Pentafluorethyl, Trifluormethoxy, Difluormethoxy, Chlordifluormethoxy, 2-Chlor-1,1,2-trifluorethoxy, 1,1,2,2-Tetrafluorethoxy, 2,2-Dichlor-1,1-difluorethoxy, 1,1,2,3,3,3-Hexafluorpropoxy, Trifluormethylthio, Difluormethylthio, Chlordifluormethylthio, 2-Chlor-1,1,2-trifluorethylthio, Methoxycarbonyldifluormethylthio, 1,1,2,3,3,3-Hexafluorpropylthio, Trifluormethylsulfonyl, Methoxy-, Ethoxy-, n-Propoxy-, i-Propoxy-, n-Butoxy-, i-Butoxy-, sec-Butoxy- und tert.Butoxycarbonyl oder für gegebenenfalls durch Halogen, Nitro, Cyano, $C_1$–$C_6$-Halogenalkyl oder $C_1$–$C_4$-Alkoxycarbonyl substituiertes Phenoxy steht, oder

$R^1$ und $R^2$ gemeinsam für Difluormethylendioxy, für $-CF_2-O-CF_2-O-$ oder für Ethylendioxy, welches durch 3 oder 4 Fluoratome oder durch 3 Fluoratome und 1 Chloratom substituiert ist, stehen,

$R^3$ für Wasserstoff, Fluor, Chlor oder Brom steht, und

$R^4$ für Wasserstoff, Chlor, Brom, Trifluormethyl, Trifluormethoxy und Trifluormethylthio steht.

3. Verbindungen gemäss Anspruch 1, wobei

X für Sauerstoff steht,

$R^1$ für Wasserstoff oder Chlor steht,

$R^2$ für Wasserstoff, Trifluormethyl, Trifluormethoxy, Difluormethoxy, Chlordifluormethoxy, Chlortrifluorethoxy, n-Pentafluorpropoxy, Trifluormethylthio, Chlordifluormethylthio, tert.Butoxycarbonyl, 4-Nitrophenoxy, 4-Cyanophenoxy und 4-Trifluormethylphenoxy steht, oder

$R^1$ und $R^2$ gemeinsam für Chlortrifluorethylendioxy oder für $-CF_2-O-CF_2-O-$ stehen,

$R^3$ für Wasserstoff oder Chlor steht, und

$R^4$ für Wasserstoff steht.

4. Verfahren zur Herstellung von 2,5-Dihalogenbenzoyl-(thio)harnstoffen der Formel (I)

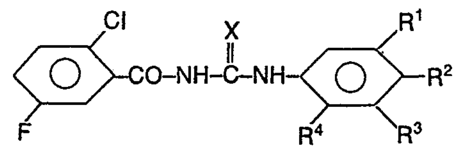

(I)

in welcher

X für Schwefel oder Sauerstoff steht,

$R^1$ für Wasserstoff, Halogen oder für einen gegebenenfalls durch Halogen substituierten Rest aus der Reihe $C_1$–$C_6$-Alkyl, $C_1$–$C_6$-Alkoxy und $C_1$–$C_6$-Alkylthio steht,

$R^2$ für Wasserstoff, Cyano, Nitro, Halogen oder für einen gegebenenfalls durch Halogen substituierten Rest aus der Reihe $C_1$–$C_6$-Alkyl, $C_1$–$C_6$-Alkylthio, $C_1$–$C_6$-Alkylsulfonyl, $C_1$–$C_6$-Alkoxy, $C_2$–$C_6$-Alkylcarbonyl, $C_2$–$C_6$-Alkoxycarbonyl, $C_3$–$C_6$-Alkoxycarbonylalkyl, $C_3$–$C_6$-Alkoxycarbonylalkylthio oder für einen gegebenenfalls durch Halogen, Cyano, Nitro, Phenyl, Trifluormethyl, Trifluormethoxy, Trifluormethylthio, $C_1$–$C_6$-Alkyl, $C_1$–$C_6$-Alkoxy, $C_1$–$C_6$-Alkylthio, $C_1$–$C_6$-Alkylthioalkyl, $C_1$–$C_6$-Alkylsulfonyloxy und/oder $C_2$–$C_6$-Alkoxycarbonyl substituiertes Phenyloxy steht, oder

$R^1$ und $R^2$ gemeinsam für gegebenenfalls durch Fluor und/oder Chlor substituiertes Alkylen-dioxy mit 1 bis 3 Kohlenstoffatomen oder für $-CF_2-O-CF_2-O-$ stehen,

$R^3$ für Wasserstoff, Halogen oder für einen gegebenenfalls durch Halogen substituierten $C_1$–$C_6$-Alkyl-, $C_1$–$C_6$-Alkoxy- oder Phenyloxy-Rest steht, und

$R^4$ für Wasserstoff, Halogen oder für gegebenenfalls durch Halogen substituierte Reste aus der Reihe $C_1$–$C_6$-Alkyl, $C_1$–$C_6$-Alkylthio oder $C_1$–$C_6$-Alkoxy steht,

dadurch gekennzeichnet, dass man

a) substituierte Aniline der Formel (II)

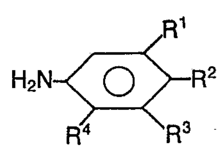

(II)

in welcher

$R^1$, $R^2$, $R^3$ und $R^4$ die oben angegebenen Bedeutungen haben,
mit Benzoyl-iso(thio)cyanaten der Formel (III)

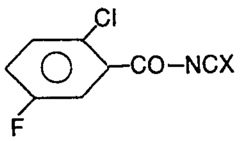

(III)

in welcher
X die oben angegebene Bedeutung hat,
gegebenenfalls in Gegenwart eines Verdünnungsmittels umsetzt oder
b) substituierte Phenyl-iso(thio)cyanate der Formel (IV)

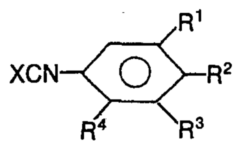

(IV)

in welcher
$R^1$, $R^2$, $R^3$, $R^4$ und die oben angegebenen Bedeutungen haben,
mit Benzoesäureamid der Formel (V)

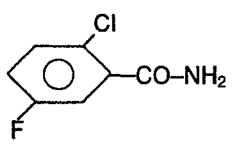

(V)

gegebenenfalls in Gegenwart eines Katalysators und gegebenenfalls in Gegenwart eines Verdünnungsmittels umsetzt.

5. Schädlingsbekämpfungsmittel, gekennzeichnet durch einen Gehalt an mindestens einer Verbindung der Formel I.

6. Verwendung von Verbindungen der Formel I zur Bekämpfung von Schädlingen, insbesondere Insekten.

7. Verfahren zur Bekämpfung von Schädlingen, dadurch gekennzeichnet, dass man Verbindungen der Formel I auf die Schädlinge, vorzugsweise Insekten oder ihren Lebensraum einwirken lässt.

8. Verfahren zur Herstellung von Schädlingsbekämpfungsmitteln, dadurch gekennzeichnet, dass man Verbindungen der Formel I mit Streckmitteln und/oder oberflächenaktiven Mitteln vermischt.

**Claims**

1. 2,5-Dihalogenobenzoyl-(thio)-ureas of the formula (I)

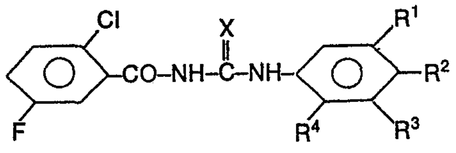

(I)

in which
X represents sulphur or oxygen,
$R^1$ represents hydrogen, halogen or an optionally halogen-substituted radical from the series comprising $C_1$–$C_6$-alkyl, $C_1$–$C_6$-alkoxy and $C_1$–$C_6$-alkylthio,
$R^2$ represents hydrogen; cyano, nitro, halogen or an optionally halogen-substituted radical from the series comprising
$C_1$–$C_6$-alkyl, $C_1$–$C_6$-alkylthio,
$C_1$–$C_6$-alkylsulphonyl, $C_1$–$C_6$-alkoxy,
$C_2$–$C_6$-alkylcarbonyl, $C_2$–$C_6$-alkoxycarbonyl,
$C_3$–$C_6$-alkoxycarbonylalkyl,
$C_3$–$C_6$-alkoxycarbonylalkylthio
or a phenyloxy optionally substituted by halogen, cyano, nitro, phenyl, trifluoromethyl, trifluoromethoxy, trifluoromethylthio,
$C_1$–$C_6$-alkyl, $C_1$–$C_6$-alkoxy, $C_1$–$C_6$-alkylthio,
$C_1$–$C_6$-alkylthioalkyl, $C_1$–$C_6$-alkylsulphonyloxy,
and/or $C_2$–$C_6$-alkoxycarbonyl, or
$R^1$ and $R^2$ together represent alkylene-dioxy which has 1 to 3 carbon atoms and is optionally substituted by fluorine and/or chlorine, or $-CF_2-O-CF_2-O-$,
$R^3$ represents hydrogen, halogen or an optionally halogen-substituted $C_1$–$C_6$-alkyl, $C_1$–$C_6$-alkoxy or phenyloxy radical, and
$R^4$ represents hydrogen, halogen or optionally halogen-substituted radicals from the series comprising $C_1$–$C_6$-alkyl, $C_1$–$C_6$-alkylthio and $C_1$–$C_6$-alkoxy.

2. Compounds according to Claim 1, wherein
X represents oxygen or sulphur,
$R^1$ represents hydrogen, fluorine, chlorine or bromine,
$R^2$ represents
hydrogen, chlorine, bromine, fluorine, cyano, nitro, methyl, ethyl, n-propyl, i-propyl, n-butyl, i-butyl, sec.-butyl, tert.-butyl, trifluoromethyl, difluoromethyl, 1,1-difluoroethyl, 1,1,2,2,2-pentafluoroethyl, trifluoromethoxy, difluoromethoxy, chlorodifluoromethoxy, 2-chloro-1,1,2-trifluoroethoxy, 1,1,2,2-tetrafluoroethoxy, 2,2-dichloro-1,1-difluoroethoxy, 1,1,2,3,3,3-hexafluoropropoxy, trifluoromethylthio, difluoromethylthio, chlorodifluoromethylthio, 2-chloro-1,1,2-trifluoroethylthio, methoxycarbonyldifluoromethylthio, 1,1,2,3,3,3-hexafluoropropylthio, trifluoromethylsulphonyl, methoxy-, ethoxy-, n-propoxy-, i-propoxy-, n-butoxy-, i-butoxy-, sec-butoxy- and tert.butoxycarbonyl
or phenoxy
which is optionally substituted by halogen, nitro, cyano, $C_1$–$C_6$-halogenoalkyl or $C_1$–$C_4$-alkoxycarbonyl, or
$R^1$ and $R^2$ together represent difluoromethylenedioxy, $-CF_2-O-CF_2-O-$ or ethylendioxy, which is substituted by 3 or 4 fluorine atoms or by 3 fluorine atoms and 1 chlorine atom,
$R^3$ represents hydrogen, fluorine, chlorine or bromine, and
$R^4$ represents hydrogen, chlorine, bromine, trifluoromethyl, trifluoromethoxy and trifluoromethylthio.

3. Compounds according to Claim 1, wherein
X represents oxygen,
$R^1$ represents hydrogen or chlorine,

$R^2$ represents hydrogen, trifluoromethyl, trifluoromethoxy, difluoromethoxy, chlorodifluoromethoxy, chlorotrifluoroethoxy, n-pentafluoropropoxy, trifluoromethylthio, chlorodifluoromethylthio, tert.butoxycarbonyl, 4-nitrophenoxy, 4-cyanophenoxy and 4-trifluoromethylphenoxy, or

$R^1$ and $R^2$ together represent chlorotrifluoroethylenedioxy or $-CF_2-O-CF_2-O-$,

$R^3$ represents hydrogen or chlorine and

$R^4$ represents hydrogen.

4. Process for the preparation of 2,5-dihalogenobenzoyl-(thio)ureas of the formula (I)

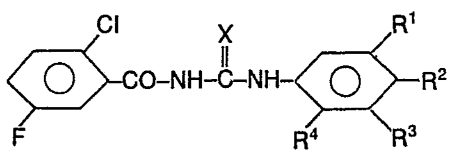 (I)

in which

X represents sulphur or oxygen,

$R^1$ represents hydrogen, halogen or an optionally halogen-substituted radical from the series comprising $C_1$–$C_6$-alkyl, $C_1$–$C_6$-alkoxy and $C_1$–$C_6$-alkylthio,

$R^2$ represents hydrogen, cyano, nitro, halogen or an optionally halogen-substituted radical from the series comprising
$C_1$–$C_6$-alkyl, $C_1$–$C_6$-alkylthio,
$C_1$–$C_6$-alkylsulphonyl, $C_1$–$C_6$-alkoxy,
$C_2$–$C_6$-alkylcarbonyl, $C_2$–$C_6$-alkoxycarbonyl,
$C_3$–$C_6$-alkoxycarbonylalkyl,
$C_3$–$C_6$-alkoxycarbonylalkylthio
or a phenyloxy optionally substituted by halogen, cyano, nitro, phenyl, trifluoromethyl, trifluoromethoxy, trifluoromethylthio, $C_1$–$C_6$-alkyl, $C_1$–$C_6$-alkoxy, $C_1$–$C_6$-alkylthio, $C_1$–$C_6$-alkylthioalkyl, $C_1$–$C_6$-alkylsulphonyloxy and/or $C_2$–$C_6$-alkoxycarbonyl, or

$R^1$ and $R^2$ together represent alkylene-dioxy which has 1 to 3 carbon atoms and is optionally substituted by fluorine and/or chlorine, or $-CF_2-O-CF_2-O-$,

$R^3$ represents hydrogen, halogen or an optionally halogen-substituted $C_1$–$C_6$-alkyl, $C_1$–$C_6$-alkoxy or phenyloxy radical, and

$R^4$ represents hydrogen, halogen or optionally halogen-substituted radicals from the series comprising $C_1$–$C_6$-alkyl, $C_1$–$C_6$-alkylthio and $C_1$–$C_6$-alkoxy, characterised in that

a) substituted anilines of the formula (II)

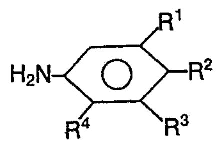 (II)

in which

$R^1$, $R^2$, $R^3$ and $R^4$ have the abovementioned meanings, are reacted with benzoyl-iso(thio)cyanates of the formula (III)

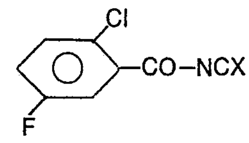 (III)

in which

X has the abovementioned meaning, if appropriate in the presence of a diluent or

b) substituted phenyl-iso(thio)cyanates of the formula (IV)

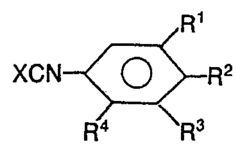 (IV)

in which

$R^1$, $R^2$, $R^3$, $R^4$ and X have the abovementioned meanings, are reacted with benzoic acid amide of the formula (V)

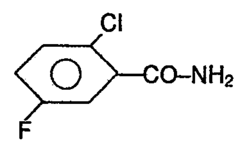 (V)

if appropriate in the presence of a catalyst and if appropriate in the presence of a diluent.

5. Agents for combating pests, characterised in that they contain at least one compound of the formula I.

6. Use of compounds of the formula I for combating pests, in particular insects.

7. Method of combating pests, characterised in that compounds of the formula I are allowed to act on the pests, preferably insects or their habitat.

8. Process for the preparation of agents for combating pests, characterised in that compounds of the formula I are mixed with extenders and/or surface-active agents.

**Revendications**

1. 2,5-dihalogénobenzoyl-(thio)-urées de formule (I)

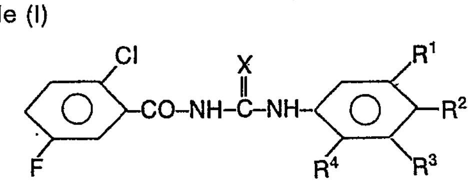 (I)

dans laquelle

X représente le soufre ou l'oxygène,

$R^1$ représente l'hydrogène, un halogène ou un reste alkyle en $C_1$–$C_6$, alcoxy en $C_1$–$C_6$ ou alkylthio en $C_1$–$C_6$, éventuellement halogéno-substitué,

$R^2$ représente l'hydrogène, cyano, nitro, un halogène ou un reste alkyle en $C_1$–$C_6$, alkylthio en $C_1$–$C_6$, alkylsulfonyle en $C_1$–$C_6$, alcoxy en $C_1$–$C_6$, alkylcarbonyle en $C_2$–$C_6$, alcoxycarbonyle en $C_2$–$C_6$, alcoxycarbonylalkyle en $C_3$–$C_6$ ou alcoxycarbonylakylthio en $C_3$–$C_6$, éventuellement substitué par un halogène, ou bien un reste phényloxy, éventuellement substitué par halogène, cyano, nitro, phényle, trifluorométhyle, trifluorométhoxy,

trifluorométhylthio, alkyle en $C_1$–$C_6$, alcoxy en $C_1$–$C_6$, alkylthio en $C_1$–$C_6$, alkylthioalkyle en $C_1$–$C_6$, alkylsulfonyloxy en $C_1$–$C_6$ et/ou alcoxycarbonyle en $C_2$–$C_6$, ou bien

$R^1$ et $R^2$ représentent ensemble un groupe alkylènedioxy en $C_1$–$C_3$ éventuellement substitué par le fluor et/ou le chlore ou un groupe $-CF_2-O-CF_2-O-$,

$R^3$ représente l'hydrogène, un halogène ou un reste alkyle en $C_1$–$C_6$, alcoxy en $C_1$–$C_6$ ou phényloxy, éventuellement substitué par un halogène, et

$R^4$ représente l'hydrogène, un halogène ou un reste alkyle en $C_1$–$C_6$, alkylthio en $C_1$–$C_6$ ou alcoxy en $C_1$–$C_6$, éventuellement substitué par un halogène.

2. Composés selon la revendication 1, dans lesquels

X représente l'oxygène ou le soufre,

$R^1$ représente l'hydrogène, le fluor, le chlore ou le brome,

$R^2$ représente
l'hydrogène, le chlore, le brome, le fluor
ou un groupe cyano, nitro, méthyle, éthyle,
n-propyle, i-propyle, n-butyle, i-butyle,
sec-butyle, tert-butyle, trifluorométhyle,
difluorométhyle, 1,1-difluoroéthyle,
1,1,2,2,2-pentafluoroéthyle, trifluorométhoxy,
difluorométhoxy, chlorodifluorométhoxy,
2-chloro-1,1,2-trifluoroéthoxy,
1,1,2,2-tétrafluoroéthoxy,
2,2-dichloro-1,1-difluoroéthoxy,
1,1,2,3,3,3-hexafluoropropoxy,
trifluorométhylthio, difluorométhylthio,
chlorodifluorométhylthio,
2-chloro-1,1,2-trifluoroéthylthio,
méthoxycarbonyldifluorométhylthio,
1,1,2,3,3,3-hexafluoropropylthio,
trifluorométhylsulfonyle, méthoxy-, éthoxy-,
n-propoxy-, i-propoxy, n-butoxy-, i-butoxy-,
sec-butoxy- ou
tert-butoxycarbonyle ou un groupe phényloxy, éventuellement substitué par halogène, nitro, cyano, halogénoalkyle en $C_1$–$C_6$ ou (alcoxy en $C_1$–$C_4$)-carbonyle, ou bien

$R^1$ et $R^2$ pris ensemble représentent un groupe difluorométhylènedioxy, un groupe $-CF_2-O-CF_2-O-$ ou un groupe éthylènedioxy qui est substitué par 3 ou 4 atomes de fluor ou par 3 atomes de fluor et 1 atome de chlore,

$R^3$ représente l'hydrogène, le fluor, le chlore ou le brome, et

$R^4$ représente l'hydrogène, le chlore, le brome ou un groupe trifluorométhyle, trifluorométhoxy ou trifluorométhylthio.

3. Composés selon la revendication 1, dans lesquels

X représente l'oxygène,

$R^1$ représente l'hydrogène ou le chlore,

$R^2$ représente l'hydrogène ou un groupe
trifluorométhyle, trifluorométhoxy,
difluorométhoxy, chlorodifluorométhoxy,
chlorotrifluoroéthoxy, n-pentafluoropropoxy,
trifluorométhylthio, chlorodifluorométhylthio,
tert-butoxycarbonyle, 4-nitrophénoxy,
4-cyanophénoxy ou 4-trifluorométhylphénoxy,

$R^1$ et $R^2$ pris ensemble repésentent un groupe chlorotrifluoroéthylènedioxy ou un groupe $-CF_2-O-CF_2-O-$,

$R^3$ représente l'hydrogène ou le chlore, et

$R^4$ représente l'hydrogène.

4. Procédé pour la fabrication de 2,5-dihalogénobenzoyl(thio)-urées de formule (I)

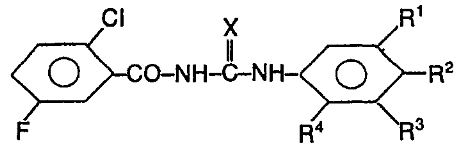
(I)

dans laquelle

X représente le soufre ou l'oxygène,

$R^1$ représente l'hydrogène, un halogène ou un reste alkyle en $C_1$–$C_6$, alcoxy en $C_1$–$C_6$ ou alkylthio en $C_1$–$C_6$, éventuellement halogéno-substitué,

$R^2$ représente l'hydrogène, cyano, nitro, un halogène ou un reste alkyle en $C_1$–$C_6$, alkylthio en $C_1$–$C_6$, alkylsulfonyle en $C_1$–$C_6$, alcoxy en $C_1$–$C_6$, alkylcarbonyle en $C_2$–$C_6$, alcoxycarbonyle en $C_2$–$C_6$, alcoxycarbonylalkyle en $C_3$–$C_6$ ou alcoxycarbonylalkylthio en $C_3$–$C_6$, éventuellement substitué par un halogène, ou bien un reste phényloxy, éventuellement substitué par halogène, cyano, nitro, phényle, trifluorométhyle, trifluorométhoxy, trifluorométhylthio, alkyle en $C_1$–$C_6$, alcoxy en $C_1$–$C_6$, alkylthio en $C_1$–$C_6$, alkylthioalkyle en $C_1$–$C_6$, alkylsulfonyloxy en $C_1$–$C_6$ et/ou alcoxycarbonyle en $C_2$–$C_6$, ou bien

$R^1$ et $R^2$ représentent ensemble un groupe alkylènedioxy en $C_1$–$C_3$ éventuellement substitué par le fluor et/ou le chlore ou un groupe $-CF_2-O-CF_2-O-$,

$R^3$ représente l'hydrogène, un halogène ou un reste alkyle en $C_1$–$C_6$, alcoxy en $C_1$–$C_6$ ou phényloxy, éventuellement substitué par un halogène, et

$R^4$ représente l'hydrogène, un halogène ou un reste alkyle en $C_1$–$C_6$, alkylthio en $C_1$–$C_6$ ou alcoxy en $C_1$–$C_6$, éventuellement substitué par un halogène,

caractérisé en ce que:

a) on fait réagir des anilines substituées de formule (II)

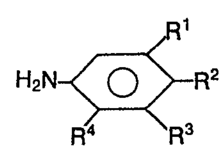
(II)

dans laquelle

$R^1$, $R^2$, $R^3$ et $R^4$ ont les significations indiquées ci-dessus, avec des iso(thio)cyanates de benzoyle de formule (III)

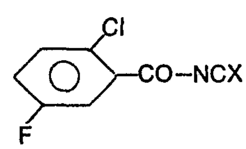
(III)

dans laquelle

X a la signification indiquée ci-dessus,

éventuellement en présence d'un agent diluant, ou bien

b) on fait réagir des iso(thio)cyanates de phényles substitués de formule (IV)

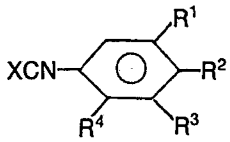

(IV)

dans laquelle

$R^1$, $R^2$, $R^3$, $R^4$ et X ont les significations indiquées ci-dessus, avec le benzamide de formule (V)

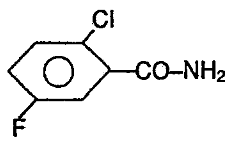

(V)

éventuellement en présence d'un catalyseur et éventuellement en présence d'un agent diluant.

5. Produits antiparasites, caractérisés en ce qu'ils contiennent au moins un composé de formule (I).

6. Utilisation de composés de formule (I) pour la lutte contre les parasites, en particulier les insectes.

7. Procédé pour la lutte contre les insectes, caractérisé en ce que l'on fait agir les composés de formule (I) sur les parasites, de préférence les insectes, ou leur espace vital.

8. Procédé pour la fabrication de produits antiparasites, caractérisé en ce que l'on mélange des composés de formule (I) avec des agents diluants et/ou des agents tensioactifs.